(19) Europäisches Patentamt
European Patent Office
Office européen des brevets

(11) Veröffentlichungsnummer: **0 203 216 A1**

(12) **EUROPÄISCHE PATENTANMELDUNG**

(21) Anmeldenummer: **85106623.3**

(22) Anmeldetag: **29.05.85**

(51) Int. Cl.[4]: **C 07 D 499/76**, C 07 D 499/68, A 61 K 31/43

(43) Veröffentlichungstag der Anmeldung: **03.12.86 Patentblatt 86/49**

(84) Benannte Vertragsstaaten: **AT BE CH DE FR GB IT LI NL SE**

(71) Anmelder: **T P O "PHARMACHIM", Iliensko Chaussée 16, Sofia (BG)**

(72) Erfinder: **Atanassova, Ivanka Assenova, Boul. Maltschika 33 Block 2-A, Plovdiv (BG)**
Erfinder: **Haimova, Marieta Avramova, Drinov Strasse 24, Sofia (BG)**
Erfinder: **Tschavdarova, Vesselina Borissova, Oborischte Strasse 103A, Sofia (BG)**
Erfinder: **Nakov, Anton Ivanov, Kiril i Metodi Strasse Block A Vchod 3 ap.14, Razgrad (BG)**
Erfinder: **Petkov, Nedeltscho Genev, Vaptzarov Strasse DNS Vchod 4, Razgrad (BG)**
Erfinder: **Avramova, Ruska Strateva, J. Kjuri Strasse 75, Sofia (BG)**

(74) Vertreter: **von Füner, Alexander, Dr. et al, Patentanwälte v. Füner, Ebbinghaus, Finck Mariahilfplatz 2 & 3, D-8000 München 90 (DE)**

(54) **Penicillin-Derivate und Verfahren zu deren Herstellung.**

(57) Es wurden neue Acylamin-Peniccilin-Antibiotika der allgemeinen Formel I synthetisiert und ein Verfahren zu deren Herstellung erarbeitet.

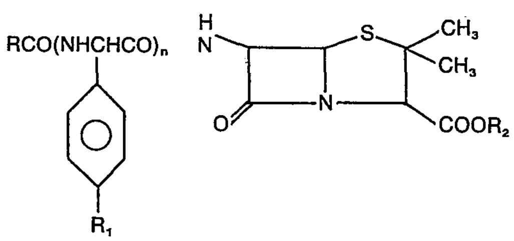

I

in welcher R die folgende Struktur aufweist:

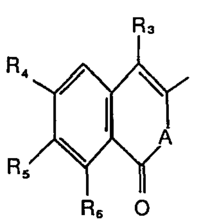

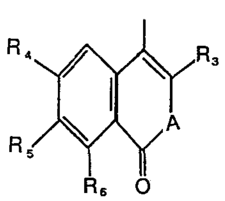

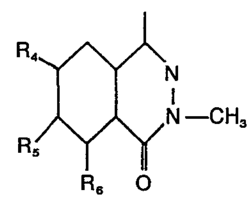

wo $R_3$ Wasserstoff, niedriger Alkylrest, Alkoxycarbonyl- oder Phenylrest bedeutet; $R_4$, $R_5$, $R_6$ bedeuten separat oder gemeinsam Wasserstoff, niedriger Alkylrest, Halogen oder niedriger Alkoxylrest; A ist ein Sauerstoffatom oder N-niedriger Alkylrest; n weist den Wert 0 oder 1 auf; $R_1$ bedeutet Wasserstoff oder eine Hydroxylgruppe und $R_2$ ist Metall oder eine Carboxy-Schutzgruppe.

Diese Antibiotika weisen eine hohe antimikrobielle Aktivität gegen gram-positive and gram-negative Mikroorganismen auf.

EP 0 203 216 A1

v. FÜNER EBBINGHAUS FINCK
PATENTANWÄLTE EUROPEAN PATENT ATTORNEYS
MARIAHILFPLATZ 2 & 3, MÜNCHEN 90
POSTADRESSE: POSTFACH 95 01 60, D-8000 MÜNCHEN 95

T P O "PHARMACHIM"

EPAA-32842.7
29. Mai 1985

# PENICILLIN-DERIVATE UND VERFAHREN ZU DEREN HERSTELLUNG

Die Erfindung betrifft Penicillin-Derivate mit pharmazeutischer Aktivität und ein Verfahren zu deren Herstellung.

Es sind Penicillin-Derivate als Derivate des Ampicillins bekannt, in denen die Aminogruppe mit Isocumarin-3-carbon-Säuren (1) und 1(2H)-Phthalazinon-4-carbon-Säuren (2) acyliert ist, welche eine Aktivität gegen Ps. aeruginosa zeigen. Aufgabe der Erfindung war es nun, neue Penicillin-Derivate mit hoher antimikrobieller Wirkung gegen gram-positive und gram-negative Mikroorganismen und ein Verfahren zu deren Herstellung zu erarbeiten.

Es sind erfindungsgemäß neue Acylamin-Penicillin-Antibiotika mit allgemeiner Formel I synthetisiert

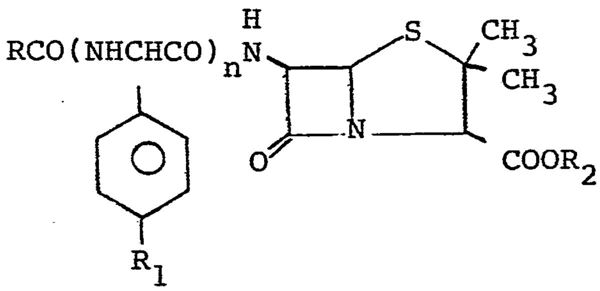

I

in welcher R folgende Struktur aufweist:

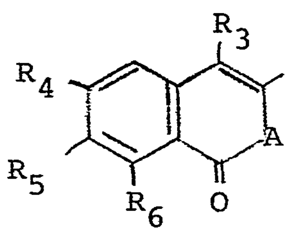
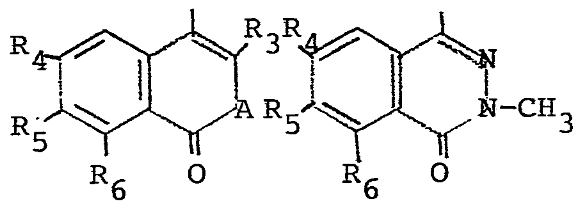

wo $R_3$ Wasserstoff, niederer Alkylrest, niederer Alkoxycarbonylrest oder Phenylrest ist; $R_4$, $R_5$, $R_6$ separat oder zusammen Wasserstoff, niederer Alkylrest, Halogen oder niederer Alkoxylrest bedeuten; A ist ein Sauerstoffatom oder N-niederer Alkylrest; n nimmt den Wert 0 oder 1 ein; $R_1$ bedeutet Wasserstoff oder Hydroxylgruppe und $R_2$ ist Wasserstoff, Alkalimetall oder eine Carboxy-Schutzgruppe.

Die hergestellten neuen Acylamin-Penicillin-Derivate wurden durch infrarote, $^1$H-kernmagnetische Spektroskopie, Elementar-Analyse und von Massenspektren identifiziert.

Die Verbindungen der Formel I werden mittels Acylierens der Aminogruppe der Penams der allgemeinen Formel II hergestellt:

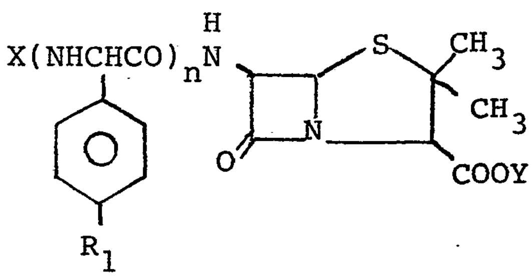

II

in welcher $R_1$ und n die oben erwähnten Bedeutungen aufweisen, X bedeutet Wasserstoff oder Trialkyl-Silylgruppe, Y ist Metall, Ammonium- oder Trialkyl-Silylgruppe, mit einem acylierenden Agens der allgemeinen Formel III

R-CO-Z III

in der R die oben erwähnten Bedeutungen aufweist, während Z -O-CO-Alk oder Halogen bedeutet.

Die acylierenden Agenzien der Formel III werden hergestellt aus 4-Methyl-3-carboxyisocumarin, 7,8-Dimethoxy-3-carboxyisocumarin, synthetisiert gemäß (3), 4-Methoxycarbonyl-3-carboxyisocumarin, synthetisiert gemäß (4), 4-Carboxyisocumarin, synthetisiert gemäß (5), 2-Methyl-4-carboxy-1(2H)-isochinolinon, synthetisiert gemäß (6) und 2-Methyl-4-carboxy-1(2H)-phthalazinon, synthetisiert gemäß (7).

Das Acylieren der Aminogruppe in den Penam-Derivaten wird gemäß der in der Chemie der Betalaktam-Antibiotika angewandten Verfahren durchgeführt (8). Die Kondensierung der Acyl-Derivate der allgemeinen Formel III mit den Penam-Derivaten in wasserfreiem oder wässerig-organischem Medium erfolgt in Anwesenheit von schwachen organischen oder anorganischen Basen als den Chlorwasserstoff bindenden Mitteln und bei einer Temperatur des Reaktionsmediums von -20 bis +20°C.

Für die Arbeit in wässerig-organischem Medium können als Lösungsmittel Aceton, Tetrahydrofuran, Dioxan, Dimethylformamid, Dimethylacetamid oder Dimethylsulphoxid verwendet werden. Zu bevorzugen ist, daß das Acylieren in sich mit dem Wasser nicht vermischbaren organischen Lösungsmitteln, wie chlorierten Kohlenwasserstoffen - Chloroform, Methylenchlorid und anderen vorgenommen wird.

Als Chlorwasserstoff-Bindemittel werden schwache organische und anorganische Basen wie Natrium-bicarbonat, N,N-Dimethylanilin, Triethylamin, Pyridin und andere verwendet.

In den Fällen, in denen das Acylieren in einem wasserfreien Medium durchgeführt wird, werden die 6-Aminopenicillan-Säure und ihre Alpha-Aminoderivate in die Reaktion in Form von N,O-Bisteralkylsilyl-Derivaten eingeführt.

Das Silylieren wird in wasserfreien organischen Lösungsmitteln durchgeführt. Als silylierende Agenzien werden Trialkylchlorsilane, Hexamethyldisilazane, N,O-Bistrimethylsilylacetamide, N,N-Bistrimethylsilylcarbamide, sowie auch eine Kombination aus Hexamethyldisilazanen und Trimethylchlorsilanen verwendet.

Das Isolieren der Endprodukte wird in Abhängigkeit von den individuellen Eigenschaften wie folgt durchgeführt: Die Metallsalze werden vom Reaktionsgemisch nach dem Extrahieren des Antibiotikums aus dem organischen Lösungsmittel mit einer Natriumbicarbonat-Lösung und Lyophilisieren oder durch ihre Fällung in organischen Lösungsmitteln mit geeigneten Fällungsagenzien isoliert (Natriumacetet, Natriumsalz der Diethylcapronsäure u.a.).

Die neuen Verbindungen zeigen eine Aktivität in vitro gegenüber pathogenen Mikroorganismen wie Staph. aureus, E. coli, Ps. vulgaris. Anhand der nachstehend angeführten Beispiele wird die Erfindung näher erläutert.

Beispiel 1

Natriumsalze der 6-(4'-Methyl-isocumarin-3'-carboxamid)-2,2-dimethyl-penam-3-carbonsäure

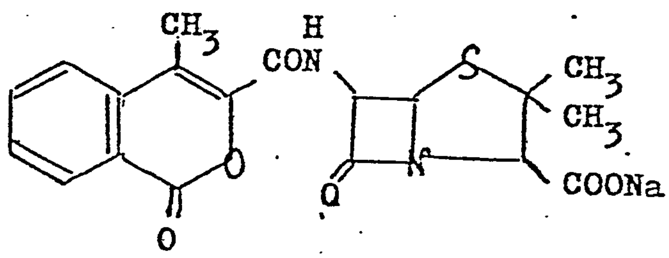

Ein mmol (216 mg) 6 Aminopenicillan-Säure und 0,23 ml (1,1 mmol) Hexamethyldisilazan und 15,0 ml Methylenchlorid werden bis zu ihrer gänzlichen Auflösung gekocht. Das Reaktionsgemisch wird bis auf -5°C abgekühlt; es werden 1 mmol (0,14 ml) Triethylamin zugegeben und danach portionsweise hartes saures Chlorid, hergestellt aus 1 mmol (204 mg) 4-Methyl-isocumarin-3-carbon-Säure und 3,0 ml Thionylchlorid. Das Abkühlen wird eingestellt und das Reaktionsgemisch wird während 1 Stunde bei Zimmertemperatur gemischt. Das Lösungsmittel wird unter Vakuum bei 40°C entfernt. Zum Rückstand werden 10,0 ml Ethylacetat beigefügt und das Gemisch auf -5°C abgekühlt. Es werden 2,0 ml Wasser zugegeben und danach die Lösung mit konzentrierter Salzsäure bis zu einem pH-Wert 2 angesäuert. Beide Schichten werden getrennt und nach dem Trocknen der organischen Schicht (Natriumsulfat) wird eine äquivlaente Menge gesättigter Alkohollösung des Natriumacetats zugegeben. Der Niederschlag von Natriumsalz wird filtriert und mit Aceton gewaschen. Die Ausbeute beträgt 290 mg (68 %), der Schmelzpunkt 178 bis 181°C (mit Zersetzung).

IR-Spektrum (Nujol, $cm^{-1}$):1600 ($COO^-$), 1650 (CO, Amid), 1730 (CO, Lakton) mit Inflex bei 1780 (CO, ß-Laktam), 3400 (NH).

Beispiel 2 bis 7

Von 216 mg Aminopenicillansäure wurden nach dem in Beispiel 1 beschriebenen Verfahren folgende Verbindungen synthetisiert, welche in Tabelle 1 veranschaulicht sind.

Tabelle 1

| Beispiel Nr. | Verbindung | Ausbeute | Schmelzpunkt, °C (mit Zersetzung) | IR-Spektrum (Nujol, $cm^{-1}$) |
|---|---|---|---|---|
| 1 | 2 | 3 | 4 | 5 |
| 2 | Natriumsalz der 6-(7',-8'-Dimethoxy-isocumarin-3'-carboxamid)-2,2-dimethyl-penam-3-carbonsäure 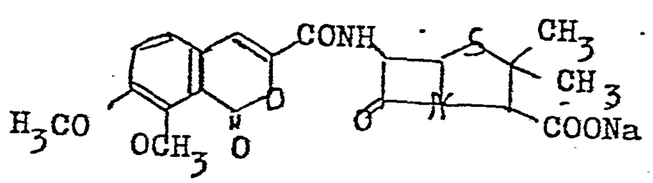 | 64 | 212-214 | 1560 ($COO^-$), 1690 (Co, Amid), 1770-breites (CO, Lakton, CO, Betalaktam), 3300 (NH) |

Tabelle 1 (Fortsetzung)

| 1 | 2 | 3 | 4 | 5 |
|---|---|---|---|---|
| 3 | Natriumsalz der (-(4'-Methoxy-carbonyl-3'-carboxamid)-2,2-dimethyl-penam-3-carbonsäure 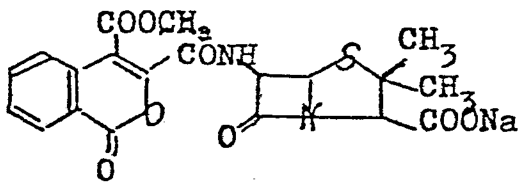 | 61 | 176-178 | 1580 ($COO^-$), 1650 (CO-Amid), 1700 (CO, Ester), 1760-breites mit Inflex, 1780 (CO, Lakton, CO, Betalaktam), 3400 (NH) |
| 4 | Natriumsalz der 6-(Isocumarin-4'-carboxamid)-2,2-dimethyl-penam-3-carbonsäure 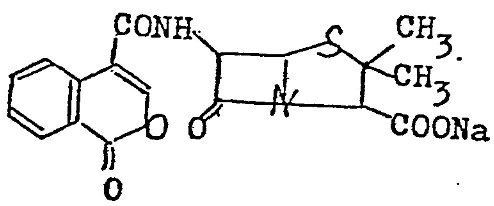 | 59 | 195-199 | 1590 ($COO^-$), 1660 (CO, Amid), 1720 (CO, Lakton), 1780 (CO, Betalaktam), 3400 (NH) |
| 5 | Natriumsalz der 6-(3'-Methyl-isocumarin-4'-carboxamid)-2,2-dimethyl-penam-3-carbonsäure 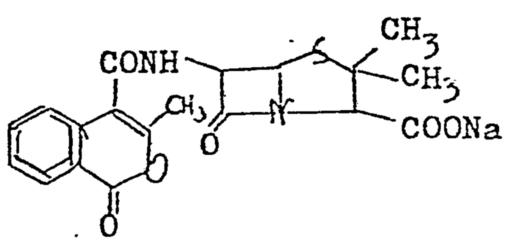 | 65 | 220-222 | 1580 ($COO^-$), 1660-breites (CO, Amid), 1750-breites (CO, Lakton, CO, Betalaktam), 3400 (NH) |

Tabelle 1 (Fortsetzung)

| 1 | 2 | 3 | 4 | 5 |
|---|---|---|---|---|
| 6 | Natriumsalz der 6-/2'-Methyl-1'(2H)-isochinolinon-4'-carboxamid/-2,2-dimethyl-penam-3-carbonsäure | 61 | 218-220 | 1600 ($COO^-$), 1660 (CO, Amid), 1780 (CO, Betaaktam), 3400 (NH) |
| | 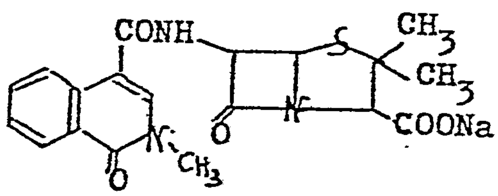 | | | |
| 7. | Natriumsalz der 6-/2'-Methyl-1'(2H)-phthalazinon-4'-carboxamid/-2,2-dimethyl-penam-3-carbonsäure | 64 | 205-210 | 1600 ($COO^-$), 1640 u. 1660 (CO, Amid), 1780 (CO, Beta-Laktam), 3400 (NH) |
| | 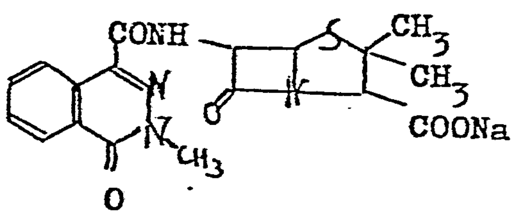 | | | |

Beispiele 8 bis 17

Aus Ampicillin (349 mg, 1 mmol) wurden folgende Verbindungen nach dem in Beispiel 1 beschriebenen Verfahren synthetisiert, welche in Tabelle 2 angegeben sind.

Tabelle 2

| Beispiel Nr. | Verbindung | Ausbeute | Schmelzpunkt, °C (Zersetzung) | IR-Spektrum (Nujol, $cm^{-1}$) |
|---|---|---|---|---|
| 8 | Natriumsalz der 6-/N-(4'-Methyl-isocumarin-3-carbonyl(-2R-2-aminophenyl-acetamid/-2,2-dimethyl-penam-3-carbonsäure 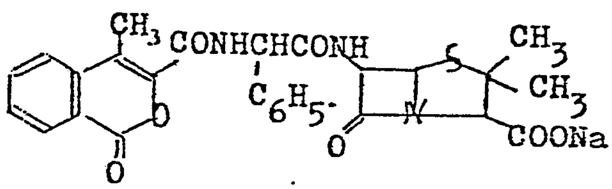 | 58 | 134-138 | 1600 ($COO^-$), 1660 (CO, Amid), 1730 (CO, Lakton), 1780 (CO, Betalaktam), 3300 (NH); jodometrische Aktivität 1023,96 U/mg (quantitativer Gehalt 96,1 %) |
| 9 | Natriumsalz der 6-/N-(7',8'-Dimethoxy-isocumarin-3'-carbonyl)-2R-2-aminophenylacetamid/-2,2-dimethyl-penam-3-carbonsäure 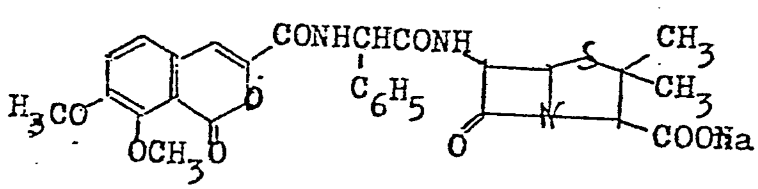 | 60 | 228-230 | 1570 ($COO^-$), 1680 (CO, Amid), 1780 (Breit, CO, Lakton und CO, Betalaktam), 3400 (NH). Jodometrische Aktivität 860,72 E/mg (quantitativer Gehalt 87,5 %) |

Tabelle 2 (Fortsetzung)

| 1 | 2 | 3 | 4 | 5 |
|---|---|---|---|---|
| 10 | Natriumsalz der 6-/N-(4'-Methoxycarbonyl-isocumarin-3'-carbonyl)-2R-2-aminophenyl-acetamid/-2,2-dimethyl-penam-3-carbonsäure<br>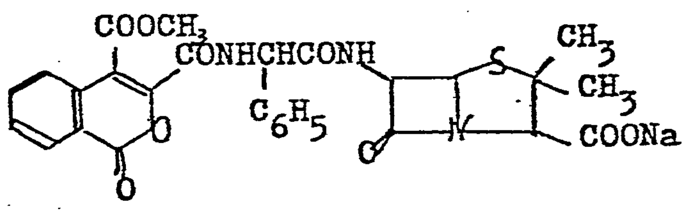 | 58 | 196÷200 | 1580 ($COO^-$), 1680 (breites CO, Amid), 1750-breit, Inflex bei 1780 (CO, Ester, Lakton und Betalaktam), 3340 (NH) Jodometrische Aktivität 905,24 E/mg (quantitativer Gehalt 91,7 %) |
| 11 | Natriumsalz der 6-/N-(Isocumarin-4'-carbonyl)-2R-2-aminophenyl-acetamid/-2,2-dimethyl-penam-3-carbonsäure<br>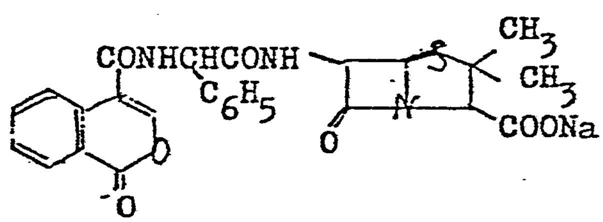 | 60 | 220-224 | 1600 ($COO^-$), 1660 (CO, Amid), 1780 (CO, Betalaktam), 3300 (NH) |
| 12 | Natriumsalz der 6-/N-(3'-Methyl-isocumarin-4'-carbonyl)-2R-2-aminophenylacetamid/-2,2- | | | 1590 ($COO^-$), 1670 (CO, Amid), 1750-breit, mit |

Tabelle 2 (Fortsetzung)

| 1 | 2 | 3 | 4 | 5 |
|---|---|---|---|---|
| | dimethyl-penam-3-carbonsäure | 58 | 186-190 | Inflex bei 1780 (CO, Lakton, CO, Betalaktam), 3400 (NH) |
| | 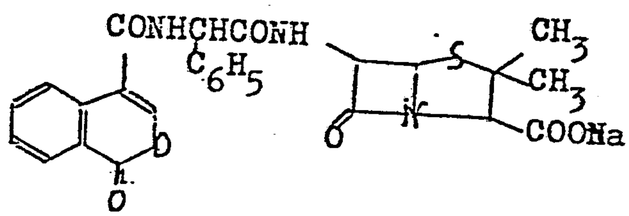 | | | |
| 13 | Natriumsalz der 6-/N-(2'-Methyl-1'/2'H/-isochinolinon-4'-carbonyl)-2R-2-aminophenylacetamid/2,2-dimethyl-penam-3-carbonsäure | 61 | 235-240 | 1590 (COO⁻), 1660 (CO, Amid), 1770 (CO, Betalaktam), 3400 (NH) |
| | 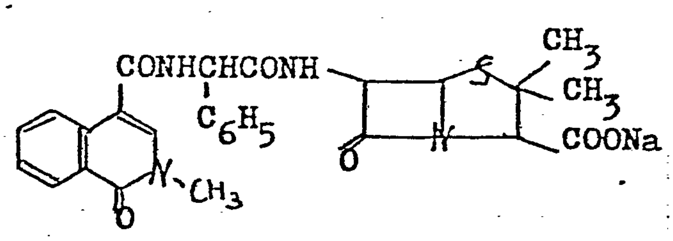 | | | |
| 14 | Natriumsalz der 6-/N-(2-Methyl-1'/2'H/-phthalazinon-4'-carbonyl/-2R-2-aminophenyl-acetamid/-2,2-dimethyl-penam-3-carbonsäure | 58 | 218-222 | 1590 (COO⁻), 1680-breit (CO, Amid), 1780 (CO, Betalaktam), 3400 (NH) |
| | 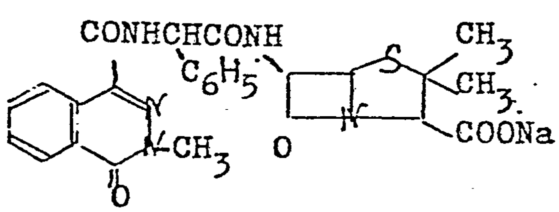 | | | |

Tabelle 2 (Fortsetzung)

| 1 | 2 | 3 | 4 | 5 |
|---|---|---|---|---|
| 15 | Natriumsalz der 6-/N-(2',4'-Dimethyl-1'/2H/-isochinolinon-3-carbonyl/-2R-2-aminophenylacetamid/-2,2-dimethyl-penam-3-carbonsäure<br>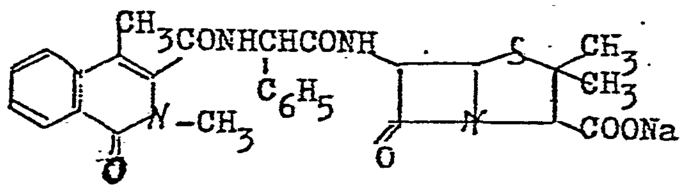 | 72 | 245-248 | 1600 ($COO^-$), 1650 (CO, Amid), 1780 (CO, Betalaktam), 3400 (NH) Jodometrische Aktivität 1009,12 E/mg (quantitativer Gehalt: 91,5 %) |
| 16 | Natriumsalz der 6-/-(4'-Phenyl-isocumarin-3'-carbonyl)-2R-2-aminophenylacetamid/-2,2-dimethyl-penam-3-carbonsäure<br>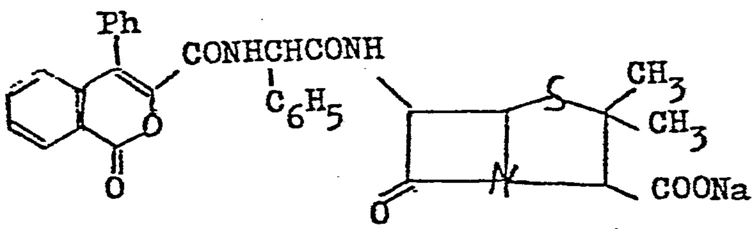 | 68 | 210-212 | 1650 (CO, Amid), 1710 (CO, Lakton), 1780 (CO, Betalaktam), 3300 (NH). Jodometrische Aktivität 985,55 E/mg (quantitativer Gehalt: 92,9 %) |
| 17 | Natriumsalz der 6-/N-(2'-Methyl-4'-phenyl-1'(2H)-isochinolinon-3'-carbonyl)-2R-2-aminophenylacetamido/-2,2-dimethyl-penam-3-carbonsäure | 65 | 215-217 | 1660 (CO, Amid), 1780 (CO, Betalaktam), 3300 (NH). Jodometrische Aktivität 938,83 E/mg (quantitativer |

Tabelle 2 (Fortsetzung)

| 1 | 2 | 3 | 4 | 5 |
|---|---|---|---|---|
| | 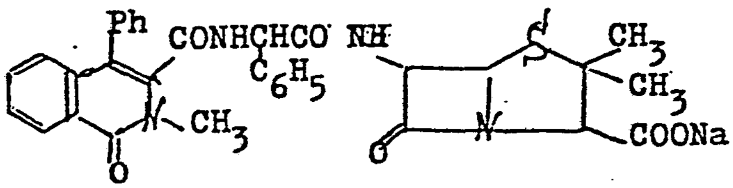 | | | Gehalt 96,4 %). |

Beispiel 18

Methylester der 6-(4'-Methyl-isocumarin-3'-carboxamid)-2,2-dimethyl-penam-carbonsäure

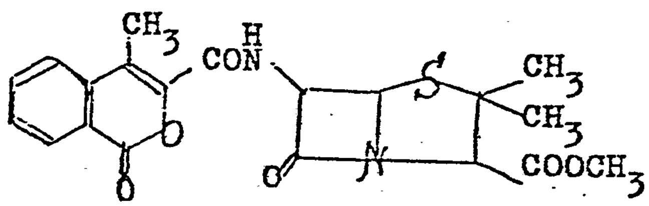

Ein mmol (216 mg) 6-Aminopenicillansäure wird mit saurem Chlorid acyliert, hergestellt aus 1 mmol (204 mg) 4-Methyl-isocumarin-3-carbonsäure, wonach das Reaktionsgemisch nach dem im Beispiel 1 angegebenen Verfahren bearbeitet wird. Zu der getrockneten Ethylacetat-Lösung des Antibiotikums wird eine Diazomethan-Etherlösung beigefügt. Nach zwei Stunden werden die Lösungsmittel unter Vakuum entfernt und der Rückstand aus Isopropanol umkristallisiert. Die Ausbeute beträgt 260 mg (63 %); der Schmelzpunkt liegt bei 161 bis 162°C.

Infrarot-Spektrum (Nujol, $cm^{-1}$): 1680 (CO, Amid), 1730 (CO, Lakton und CO, Ester), 1800 (CO, Betalaktam), 3430 (NH).

$^1$H-kernmagnetische Resonanz (Aceton-$d_6$), 100 MHz, Delta; 1,52 und 1,68/2x3H, jedes s,$C(CH_3)_2$/, 2,64 (s,3H,$CH_3$), 3,76 (s, 3H, $OCH_3$), 4,48 (s, 1H, 3-H), 5,6-5,9 (m, 5-H und (-H), 7,6-8,0 (3 arom.H), 8,0-8,3 (m, 8-H und NH).

Berechnet, %: N 6,73; S 7,68
Gefunden, %: N 6,80; S 8,01 $C_{20}H_{20}N_2O_6S$ (416,38)

Beispiel 19

Methylester der 6-(7',8'-Dimethoxy-isocumarin-3'-carboxamid)-2,2-dimethyl-penam-3-carbonsäure

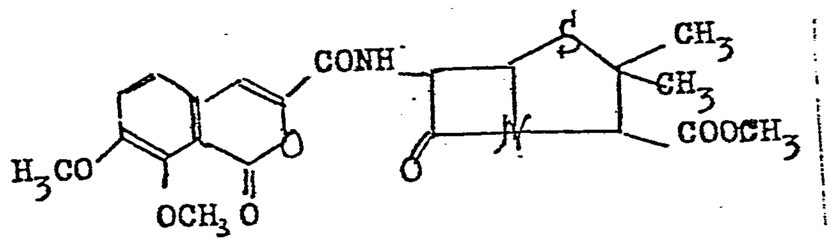

Er wird hergestellt aus 1 mmol (216 mg( 6-Aminopenicillansäure und 1 mmol (250 mg) 7,8-Dimethoxy-isocumarin-3-carbonsäure in derselben Verfahrensweise, wie im Beispiel 18. Die Ausbeute beträgt 300 mg (65 %), der Schmelzpunkt liegt bei 83 bis 85$^oC$ (aus Isopropanol). IR-Spektrum (Chloroform), $cm^{-1}$: 1690 (CO, Amid), 1750 - breites (CO, Lakton und CO, Ester), 1800 (CO, Betalaktam), 3440 (NH).

$^1$H-kernmagnetisches Spektrum (100 MHz, DMCO-$d_6$), Delta: 1,42 und 1,62 (2x3H, jedes s, $C(CH_3)_2$), 3,68 (s, 3H $OCH_3$), 3,76 (s, 3H, $OC_3$), 3,86 (s, 3H, $COOCH_3$), 4,44 (s, 1H, NH, es wird durch $D_2O$) ausgetauscht.

Berechnet, %: N 6,06; S 6,92
Gefunden, %: N 5,50; S 6,72 $C_{21}H_{22}N_2O_8S$ (462,40)

Beispiel 20

Methylester der 6-(4'Methoxycarbonyl-isocumarin-3'-carboxamid)-2,2-dimethyl-penam-carbonsäure

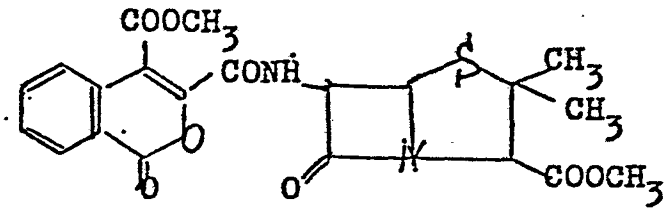

Er wird aus 1 mmol (231 mg) 4-Methoxycarbonyl-isocumarin-3'-carbonsäure und 1 mmol (216 mg) 6-Aminopenicillansäure in derselben Verfahrensweise, wie im Beispiel 18 hergestellt. Die Ausbeute beträgt 290 mg (63 %), der Schmelzpunkt liegt bei 85 bis 87°C (aus Isopropanol).

Infrarotes Spektrum (Chloroform), $cm^{-1}$: 1680 (CO, Amid), 1740 (CO, Ester und CO, Delta-Lakton), 1790 (CO, Beta-laktam), 3400 (NH).

$^{1}$H-kernmagnetisches Spektrum (80 MHz, $CDCl_3$), Delta: 1,51 und 1,72 /(2x3H, jedes s, 6H, $C(CH_3)_2$/, 3,78 (s, 3H, $COOCH_3$), 4,02 (s, 3H, $COOCH_3$), 4,50 (s, 1H, 3-H), 5,4-6,0 (m, 2H, 5-H und 6-H), 7,2-8,0 (m, 3 arom. H und NH), 8,2-8,5 (m, 1H, 8-H).

Berechnet, %: C 54,78; H 4,38; N 6,09

Gefunden, %: C 54,16; H 4,52; N 6,52 $C_{21}H_{20}N_2O_8C$ (460,39).

Beispiel 21

Methylester der 6-(Isocumarin-4'-carboxamid)-2,2-dimethyl-penam-3-carbonsäure

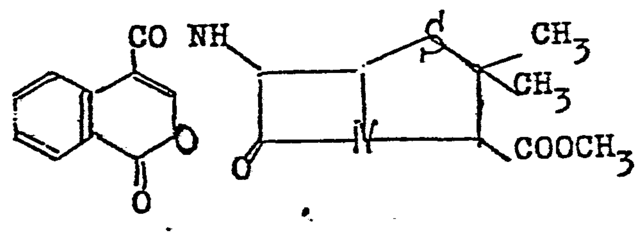

Er wird aus 1 mmol (216 mg) 6-Aminopenicillansäure und 1 mmol (190 mg) 4-Carboxyisocumarin in derselben Verfahrensweise, wie im Beispiel 18 hergestellt. Ausbeute: 250 mg (63 %), Schmelzpunkt 89 bis 92°C (aus Isopropanol).

IR-Spektrum (Nujol), $cm^{-1}$: 1690 (CO, Amid), 1740-breite (CO, Deltalakton, CO, Ester), 1800 (CO, Betalaktam), 3400 (NH).

$^{1}H$-kernmagnetisches Spektrum (Aceton-$d_6$, 100 MHz), Delta: 1,48 und 1,64/2x3H, jedes s, $C(CH_3)_2$/, 3,76 (s, 3H, $OCH_3$), 4,40 (s, 1H, 3-H), 5,6-5,8 (5-H, 6-H, m), 7,5-8,3 (m, 5 arom.H und NH).

Berechnet, %: N 6,66; S 7,61

Gefunden, %: N 6,29; S 7,45 $C_{19}H_{18}N_2O_6S.H_2O$ (420,37)

<u>Beispiel 22</u>

Methylester der 6-(3'-Methyl-isocumarin-carboxamid)-2,2-dimethyl-penam-3-carbonsäure

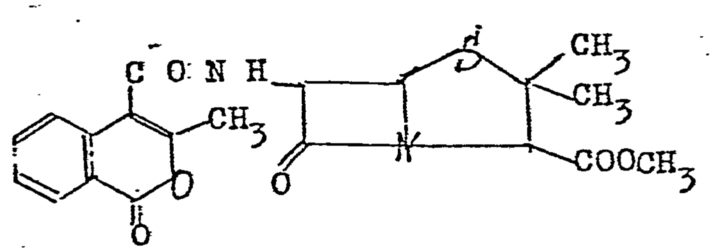

Er wird aus 1 mmol (216 mg) 6-Aminopenicillansäure und 1 mmol (204 mg) 3-Methyl-4-carboxyisocumarin in derselben Verfahrensweise, wie im Beispiel 18 hergestellt. Die Ausbeute beträgt 250 mg (60 %), der Schmelzpunkt liegt bei 88 bis 92°C (aus Isopropanol).

Infrarot-Spektrum (Chloroform), $cm^{-1}$: 1690 (CO, Amid), 1740-breite (CO, Lakton und CO, Ester), 1790 (CO, Betalaktam), 3400 (NH).

$^{1}H$-kernmagnetisches Spektrum (Aceton-$d_6$, 100 MHz), Delta: 1,44 und 1,56 /2x3H, jedes s, $C(CH_3)_2$/, 2,32 (s, 3H, $OCH_3$), 3,68 (s, 3H, $OCH_3$), 4,36 (Delta, 1H, 3-H), 5,6-5,8 (m, 2H, 5-H und 6-H; dd, $J_{5,6}$=4,5 Hz, dd nach Austausch mit $D_2O$) 7,1-8,5 (m, 4 arom. H und NH).

Berechnet, %: N 6,73; S 7,68
Gefunden, %: N 6,63; S 7,92 $C_{20}H_{20}N_2O_6S$ (416,38).

Beispiel 23

Methylester der 6-(2'-Methyl-1/2H/-isochinolinon-4'-carboxamid)-2,2-dimethyl-penam-3-carbonsäure

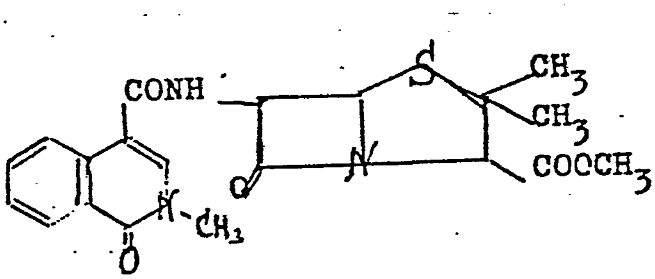

Er wird aus 1 mmol (216 mg) 6-Aminopenicillansäure und 1 mmol (203 mg) 2-Methyl-1(2H)isochinolinon-4-carbonsäure in derselben Verfahrensweise wie im Beispiel 18 hergestellt. Die Ausbeute beträgt 245 mg (59 %), der Schmelzpunkt liegt bei 118 bis 122°C (aus Isopropanol).

Infrarot-Spektrum (Chloroform, $cm^{-1}$): 1600 (CO, Amid), 1740 (CO, Ester), 1790 (CO, Betalaktam), 3400 (NH).

$^1$H-kernmagnetisches Spektrum (Aceton-$d_6$, 100 MHz): 1,44 und 1,60/2x3H jedes s, $C(CH_3)_2$/, 3,52 (s, 3H, $NCH_3$), 3,72 (s, 3H, $OCH_3$), 4,40 (s, 1H, 3H), 5,6-5,8 (m, 2H, 5-H und 6-H), 7,3-8,4 (m, 5 arom. H und NH).

Massen-Spektrum M/E: 415 ($M^+$). $C_{20}H_{21}N_3O_5S$ (415,39).

<u>Beispiel 24</u>

Methylester der 6-(2'-Methyl-1'/2'H/-phthalazinon-4'-carboxamid)-2,2-dimethyl-penam-3-carbonsäure

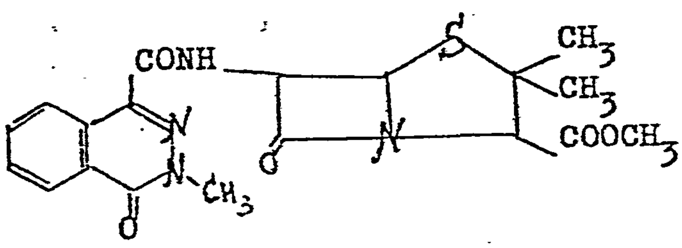

Er wird aus 1 mmol (216 mg) 6-Aminopenicillansäure und 1 mmol (204 mg ) 2-Methyl-1(2H)-phthalazinon-4-carbonsäure in derselben Verfahrensweise wie im Beispiel 18 hergestellt. Die Ausbeute beträgt 270 mg (65 %), der Schmelzpunkt liegt bei 98 bis 100°C (aus Isopropanol).

Infrarot-Spektrum (Chloroform, $cm^{-1}$): 1670-breites (CO, -Amid), 1750 (CO, Ester), 1800 (CO, Betalaktam), 3400 (NH).

$^1$H-kernmagnetisches Spektrum (Aceton-$d_6$, 100 MHz), Delta: 1,48 und 1,64 (2x3H, jedes s, C/$CH_3$/$_2$), 3,72 (Delta, 3H, $NCH_3$), 3,74 (s, 3H, $OCH_3$), 4,44 (s, 1H, 3-H), 5,6-5,8 (m, 2H, 5-H, 6-H, $J_{5,6}$ = 4,5 Hz, dd nach dem Austausch mit $D_2O$), 7,6-7,8 (m, 3 arom. H), 8,1-8,3 (m,

1H, 8-H), 8,7-8,9 (m, 1H, NH).

Berechnet, %: N 13,46; S 7,68
Gefunden, %: N 13,08; S 7,79 $C_{19}H_{20}N_4O_5S$ (416,38).

Beispiele 25 bis 32

Aus Ampicillin (349 mg) nach dem im Beispiel 15 beschriebenen Verfahren wurden folgende Verbindungen synthetisiert:

Tabelle 3

| Beispiel Nr. | Verbindung | Ausbeute % | Schmelzpunkt °C | IR-Spektrum $cm^{-1}$ | Elementaranalyse, Berechnet, % Gefunden, % |
|---|---|---|---|---|---|
| 1 | 2 | 3 | 4 | 5 | 6 |
| 25 | Methylester der 6-/N-(4'-Methylisocumarin-3'-carbonyl)-2R-2-aminophenylacetamid/-2,2-dimethyl-penam-3-carbonsäure 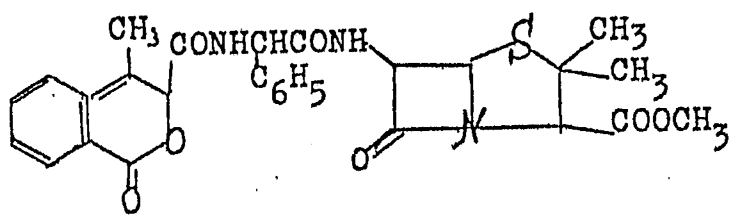 | 60 | 118-120 | (Chloroform) 1660 (CO, Amid) 1730- breites (CO, Lakton und CO, Ester), 1790 (CO, Betalaktam), 3400 (NH) | N 7,65 S 5,82<br>N 7,21 S 6,11<br>$C_{28}H_{27}N_3O_7S$ (549,52) |
| 26 | Methylester der 6-/N-(7',8'-Dimethoxy-isocumarin-3'-carbonyl)-2R-2-aminophenylacetamid/-2,2-dimethyl-penam-3-carbonsäure | 63 | 130-132 | (Chloroform) 1680 (CO, Amid), 1740- breites (CO, Ester, CO, Lakton), 1800 | N 7,06 S 5,38<br>N 6,94 S 6,14<br>$C_{29}H_{29}N_3O_9S$ (595,30) |

Tabelle 3 (Fortsetzung)

| 1 | 2 | 3 | 4 | 5 | 6 |
|---|---|---|---|---|---|
| | 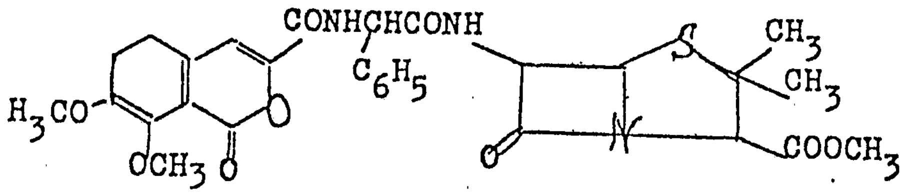 | | | (CO, Betalaktam), 3440 (NH) | |
| 27 | Methylester der 6-/N-(4'-Methoxycarbonyl-isocumarin-3'-carbonyl)-2R-2-aminophenylacetamid/-2,2-dimethyl-penam-3-carbonsäure | 62 | 143-145 | (Nujol) 1680 (CO, Amid), 1730-breite (CO, Ester und Lakton), 1790 (CO, Betalaktam), 3400 (NH) | N 7,08 S 5,39<br>N 7,20 S 5,54<br>$C_{29}H_{27}N_3O_9S$<br>(593,10) |
| | 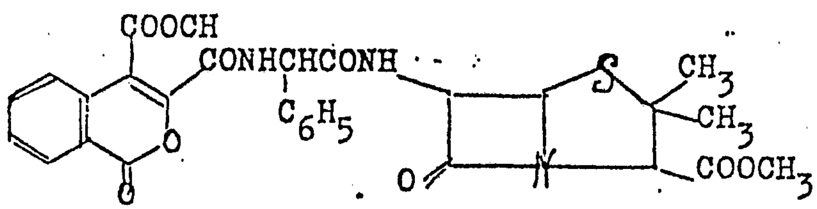 | | | | |
| 28 | Methylester der 6-/N-(Isocumarin-4'-carbonyl)-2R-2-aminophenylacetamid/-2,2-dimethyl-penam-3-carbonsäure | 63 | 103-105 | (Chloroform) 1680 (CO, Amid), 1740-breites (CO, Lakton und CO, Ester) 1800 (CO, | N 7,59 S 5,48<br>N 7,29 S 5,63<br>$C_{27}H_{25}N_3O_7S.H_2O$<br>(553,51) |

Tabelle 3 (Fortsetzung)

| 1 | 2 | 3 | 4 | 5 | 6 |
|---|---|---|---|---|---|
| | 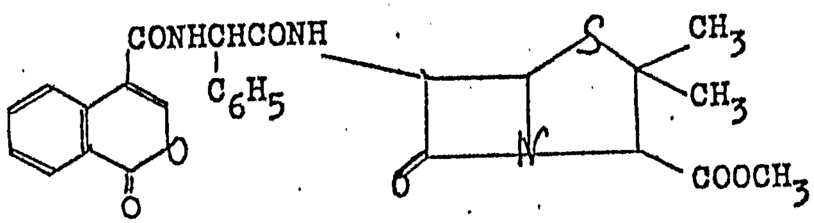 | | | Laktam) 3400 (NH) | |
| 29 | Methylester der 6-/N-(3'-Methyl-isocumarin-4'-carbonyl)-2R-2-aminophenylacetamid/-2,2-dimethyl-penam-3-carbonsäure | 60 | 104-108 | (Chloroform) 1680 (CO, Amid), 1740-breites (CO, Lakton und CO, Ester), 1790 (CO, Betalaktam), 3400 (NH) | N 7,65 S 5,82<br>N 7,26 S 5,54<br>$C_{28}H_{27}N_3O_7S$<br>(549,52) |
| | 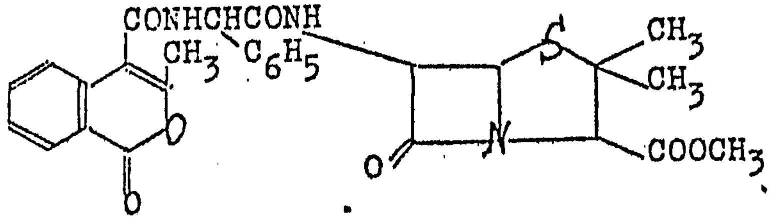 | | | | |
| 30 | Methylester der 6-/N-(2'-Methyl-1'(2H)-isochinolinon-4'-carbonyl/-2R-2-aminophenylacetamid/-2,2-dimethyl-penam-3-carbonsäure | 57 | 140-143 | (Nujol) 1660 (CO, Amid), 1740 (CO, Ester), 1790 (CO, Betalaktam) | N 10,21 S 5,84<br>N 9,87 S 6,13<br>$C_{28}H_{28}N_4O_6S$<br>(548,54) |

Tabelle 3 (Fortsetzung)

| 1 | 2 | 3 | 4 | 5 | 6 |
|---|---|---|---|---|---|
| | 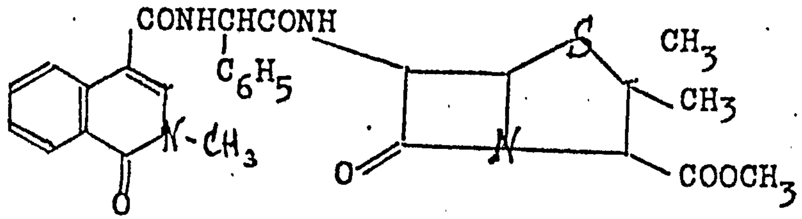 | | | 3400 (NH) | |
| 31 | Methylester der 6-/N-(2'-Methyl-1'/2H/-phthalazinon-4'-carbonyl)-2R-2-aminophenylacetamid/-2,2-dimethyl.penam-3-carbonsäure | 62 | 118-120 | (Chloroform) 1670-breites (CO, Amid), 1740 (CO, Ester), 1810 (CO, Betalaktam), 3400 (NH) | Massenspektrum M/E : 549 ($M^+$) $C_{27}H_{27}N_5O_6S$ (549,53) |
| | 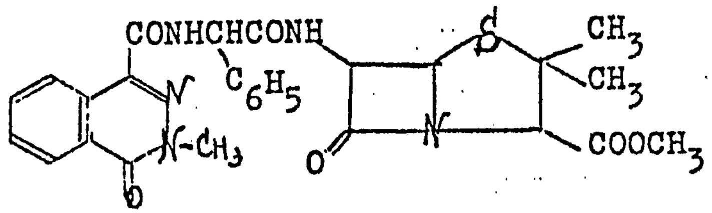 | | | | |
| 32 | Methylester der 6-/N-(2',4'-Dimethyl-1'/2H/-isochinolinon-3'-carbonyl)-2R-2-aminophenylacetamid/-2,2-dimethyl-penam-3-carbonsäure | 75 | 139-141 | (Nujol) 1650-breites (CO, Amid), 1730 (CO, Ester), 1790 (CO, Betalak- | N 9,96 S 5,69 N 9,81 S 6,15 $C_{29}H_{30}N_4O_6S$ (562,56) |

Tabelle 3 (Fortsetzung)

| 1 | 2 | 3 | 4 | 5 | 6 |
|---|---|---|---|---|---|
| 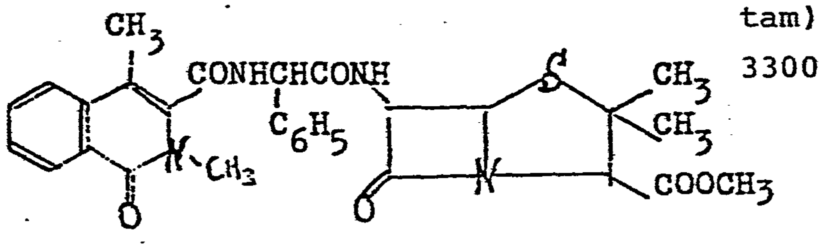 | | | | tam), 3300 (NH) | |

Beispiel 33

In der nächsten Tabelle 4 ist die Aktivität der neuen erfindungsgemäßen Verbindungen veranschaulicht. Einige der Antibiotika zeigen ein breites Wirkungsspektrum - Tabelle 5.

MIK (MIC) bedeutet in beiden Tabellen die minimale wachstumshemmende Konzentration.

Tabelle 4

Minimale Inhibierungskonzentration (mkg/ml)

| Verbindung | Stamm | | | | |
|---|---|---|---|---|---|
| | Ps. aeruginosa 2 | P. vulgaris 10 | Staph. aureus 31 | P. mirabilis 171 | E. coli II-0125 |
| 1 | 2 | 3 | 4 | 5 | 6 |
| 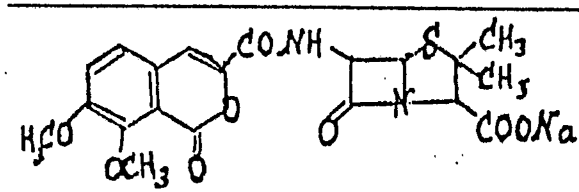 | > 100 | >100 | <12,5 | >100 | >100 |
| 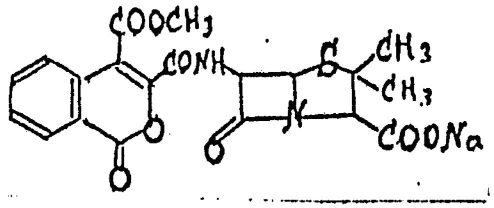 | > 100 | >100 | <12,5 | >100 | >100 |
| 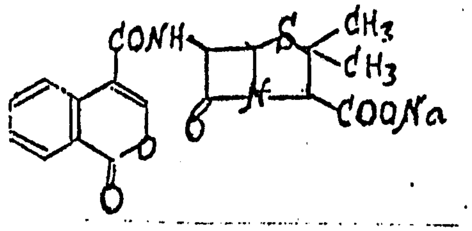 | > 100 | >100 | 12,5 | 100 | 50 |
| 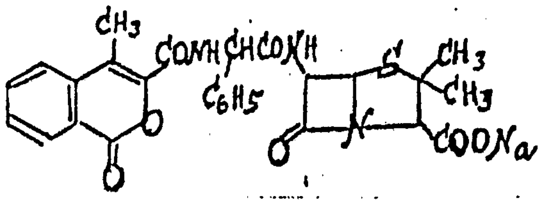 | 6,25 | >100 | <3,1 | <3,1 | <3,1 |

Tabelle 4 (Fortsetzung

| 1 | 2 | 3 | 4 | 5 | 6 |
|---|---|---|---|---|---|
| 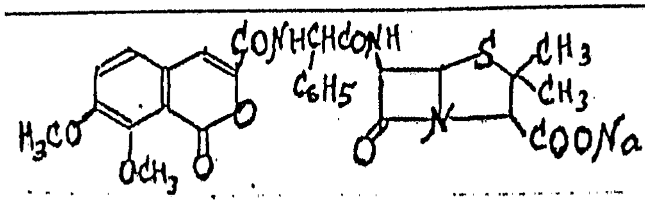 | <12,5 | >100 | <12,5 | <12,5 | <12,5 |
| 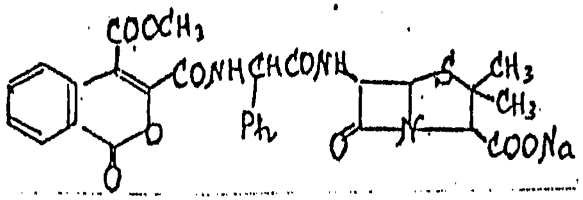 | <12,5 | >100 | <12,5 | <12,5 | <12,5 |
| 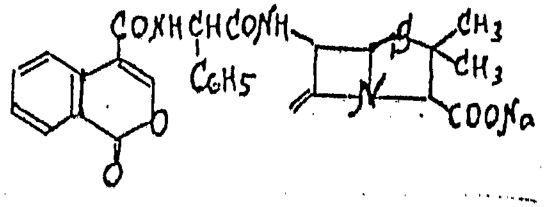 | 100 | >100 | <3,1 | 6,25 | 25 |
| 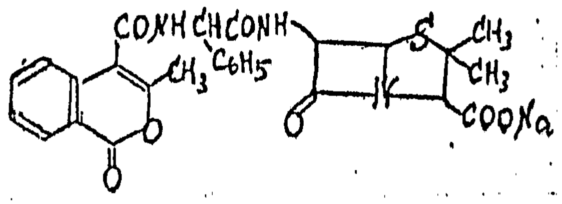 | 100 | >100 | 100 | 100 | 25 |

Tabelle 4 (Fortsetzung)

| 1 | 2 | 3 | 4 | 5 | 6 |
|---|---|---|---|---|---|
| 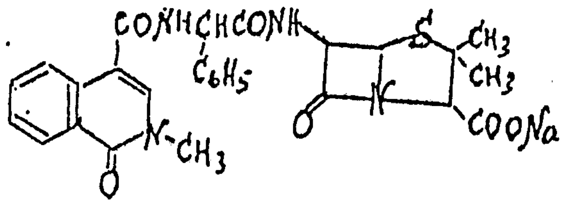 | 100 | >100 | 100 | >100 | 100 |
| 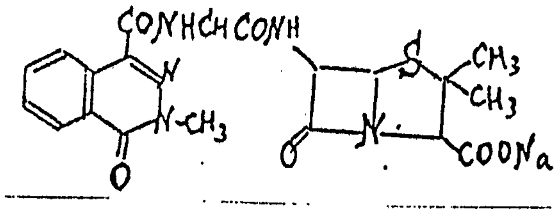 | 50 | >100 | 12,5 | 12,5 | 2,5 |
| 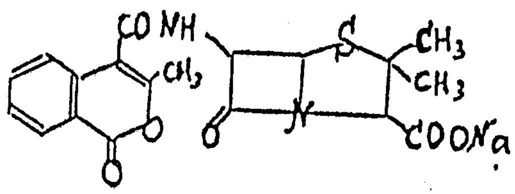 | > 100 | < 6,25 | >100 | >100 | >100 |
| 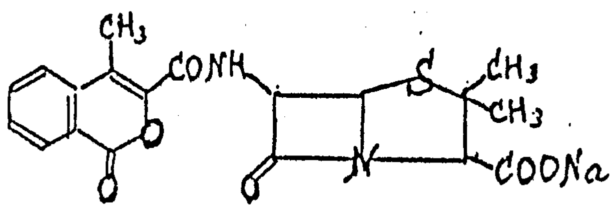 | > 100 | < 6,25 | >100 | >100 | >100 |

Tabelle 4 (Fortsetzung)

| 1 | 2 | 3 | 4 | 5 | 6 |
|---|---|---|---|---|---|
| 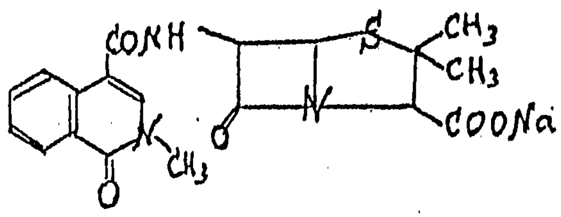 | >100 | <6,25 | >100 | >100 | >100 |
| 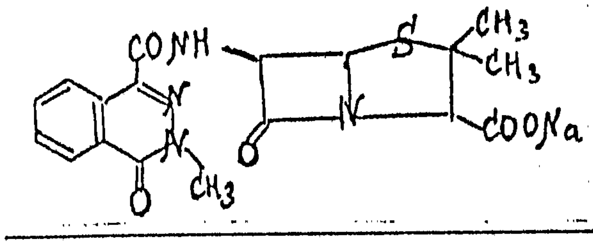 | >100 | <6,25 | >100 | >100 | >100 |

Tabelle 5

Minimale Inhibierungskonzentration (mkg/ml)

| Verbindung | Stamm | | | | | | | |
| --- | --- | --- | --- | --- | --- | --- | --- | --- |
| | Ps. aeruginosa | | | | | | Vl.pneumonia | |
| | 38 | 632 | 634 | 635 | 665 | 671 | 597 | 576 |
| 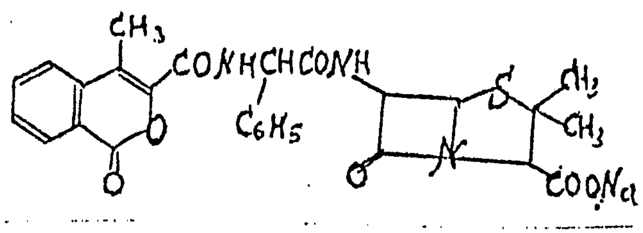 | 6,25 | | 6,25 | | | 25 | | |
| 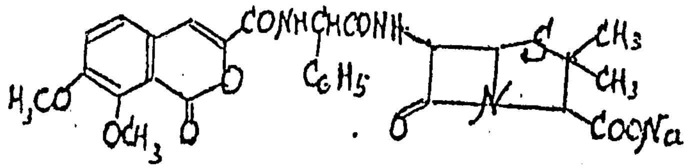 | | 6,25 | 6,25 | 25 | 25 | | 12,5 | 12,5 |
| 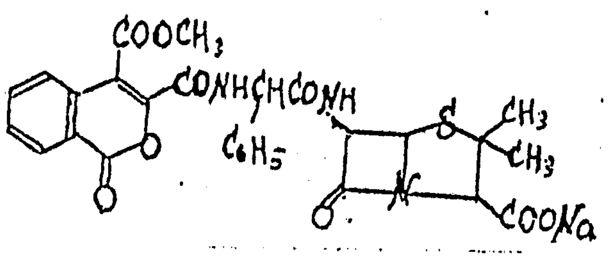 | 12,5 | 12,5 | 25 | 25 | | | | |

Tabelle 5 (Fortsetzung)

| Stamm<br>Entero. 640 | E. coli 626 | 669 | bacter 667 | P. mirabilis 660 | 661 | 662 |
|---|---|---|---|---|---|---|
| 3;1 | 12,5 | 12,5 | 12,5 | 50 | 50 | 12,5 |
| 6,25 | 6,25 | 25 | 50 | >50 | 50 | 12,5 |

# PENICILLIN-DERIVATE UND VERFAHREN ZU DEREN HERSTELLUNG

## Patentansprüche

1. Acylamin-Penicillin-Antibiotika mit allgemeiner Formel I

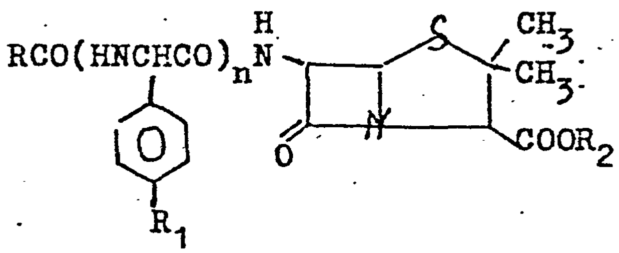

I

in der R folgende Struktur aufweist:

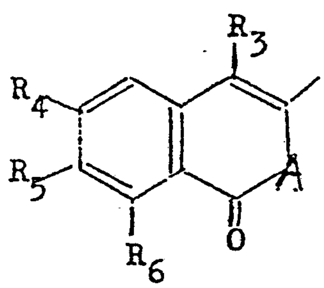

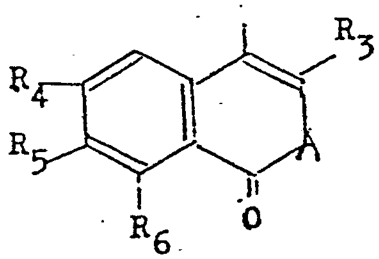

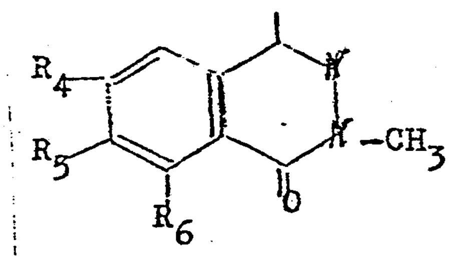

wo $R_3$ Wasserstoff, niedriger Alkylrest, Alkoxycarbonyl- oder Phenylrest bedeutet; $R_4$, $R_5$, $R_6$ bedeuten separat oder gemeinsam Wasserstoff, niedriger Alkylrest, Halogen, oder niedriger Alkoxylrest; A ist ein Sauerstoffatom oder N-niedriger Alkylrest; n weist den Wert 0 oder 1 auf; $R_1$ bedeutet Wasserstoff oder eine Hydroxylgruppe und $R_2$ ist Metall oder eine Carboxy-Schutzgruppe.

2. Natriumsalz der 6-(4'-Methyl-isocumarin-3'-carboxamid)-2,2-dimethyl-penam-3-carbonsäure.

3. Natriumsalz der 6-(7'8'-Dimethoxy-isocumarin-3'-carboxamid)-2,2-dimethyl-penam-3-carbonsäure.

4. Natriumsalz der 6-(4'-Methoxycarbonyl-isocumarin-3'-carboxamid)-2,2-dimethyl-penam-3-carbonsäure.

5. Natriumsalz der 6-(Isocumarin-4'-carboxamid)-2,2-dimethyl-penam-3-carbonsäure.

6. Natriumsalz der 6-(3'-Methyl-isocumarin-4'-carboxamid)-2,2-dimethyl-penam-3-carbonsäure.

7. Natriumsalz der 6-/2'-Methyl-1'(2'H)-isochinolinon-4'-carboxamid/-2,2-dimethyl-penam-3-carbonsäure.

8. Natriumsalz der 6-(2'-Methyl-1'/2'H/-phthalazinon-4'-carboxamid)-2,2-dimethyl-penam-3-carbonsäure.

9. Natriumsalz der 6-/N-(4'-Methyl-isocumarin-3'-carbonyl)-2R-aminophenylacetamid/-2,2-dimethyl-penam-3-carbonsäure.

10. Natriumsalz der 6-/N-(7',8'-Dimethoxy-isocumarin-3'-carbonyl)-2R-2-aminophenylacetamid/-2,2-dimethyl-penam-3-carbonsäure.

11. Natriumsalz der 6-/N-(4'-Methoxycarbonyl-isocumarin-3'-carbonyl)-2R-2-aminophenylacetamid/-2,2-dimethyl-penam-3-carbonsäure.

12. Natriumsalz der 6-/N-(Isocumarin-4'-carbonyl)-2R-2-aminophenyl-acetamid/-2,2-dimethyl-penam-3-carbonsäure.

13. Natriumsalz der 6-/N-(3'-Methyl-isocumarin-4'-carbonyl)-2R-2-aminophenylacetamid/-2,2-dimethyl-penam-3-carbonsäure.

14. Natriumsalz der 6-/N-(2'-Methyl-1'(2'H)-isochino-

linon-4'-carbonyl/-2R-2-aminophenylacetamid/-2,2-dimethyl-penam-3-carbonsäure.

15. Natriumsalz der 6-/N-(2'-Methyl-1'(2'H)-phthalazinon-4'-carbonyl/-2R-2-aminophenylacetamid/-2,2-dimethyl-penam-3-carbonsäure.

16. Methylester der 6-(4'-Methyl-isocumarin-3'-carboxamid/-2,2-dimethyl-penam-3-carbonsäure.

17. Methylester der 6-(7'8'-Dimethoxyphenyl-isocumarin-3'-carboxamid)-2,2-dimethyl-penam-3-carbonsäure.

18. Methylester der 6-(4'-Methoxycarbonyl-isocumarin-3'-carboxamid)-2,2-dimethyl-penam-3-carbonsäure.

19. Methylester der 6-(Isocumarin-4'-carboxamid)-2,2-dimethyl-penam-3-carbonsäure.

20. Methylester der 6-(3'-Methyl-isocumarin-4'-carboxamid)-2,2-dimethyl-penam-3-carbonsäure.

21. Methylester der 6-/2'-Methyl-1'(2'H)-isochinolinon-4'-carboxamid/-2,2-dimethyl-penam-3-carbonsäure.

22. Methylester der 6-/2'-Methyl-1'(2'H)-phthalazinon-4'-carboxamid)-2,2-dimethyl-penam-carbonsäure.

23. Methylester der 6-/N-(4'-Methyl-isocumarin-3'-carbonyl)-2R-2-aminophenylacetamid/-2,2-dimethyl-penam-3-carbonsäure.

24. Methylester der 6-/N-(7',8'-Dimethoxy-isocumarin-3'-carbonyl)-2R-2-aminophenylacetamid/-2,2-dimethyl-penam-3'-carbonsäure.

25. Methylester der 6-/N-(4'-Methoxycarbonyl-isocumarin-3'-carbonyl)-2R-2-aminophenylacetamid/-2,2-dimethyl-penam-3-carbonsäure.

26. Methylester der 6-/N-(Isocumarin-4'-carbonyl)-2R-2-aminophenyl-acetamid/-2,2-dimethyl-penam-3-carbonsäure.

27. Methylester der 6-/N-(3'-Methyl-isocumarin-4'-carbonyl)-2R-2-aminophenylacetamid/-2,2-dimethyl-penam-3-carbonsäure.

28. Methylester der 6-/N-(2'-Methyl-1'(2'H)-isochinolinon-4'-carbonyl)-2R-2-aminophenylacetamid/-2,2-dimethyl-penam-3-carbonsäure.

29. Methylester der 6-/N-(2'-Methyl-1'(2'H)-phthalazinon-4'-carbonyl)-2R-2-aminophenylacetamid/-2,2-dimethyl-penam-3-carbonsäure.

30. Natriumsalz der 6-/N-(2',4'-Dimethyl-1(2'H)-isochinolinon-3'-carbonyl)-2R-2-aminophenylacetamid/-2,2-dimethyl-penam-3-carbonsäure.

31. Methylester der 6-/N-(2',4'-Dimethyl-1'(2'H)-isochinolinon-3'-carbonyl)-2R-2-aminophenylacetamid/-2,2-dimethyl-penam-carbonsäure.

32. Natriumsalz der 6-/N-(4'-Phenyl-isocumarin-3'-carbonyl)-2R-2-aminophenylacetamid/-2,2-dimethyl-penam-3-carbonsäure.

33. Natriumsalz der 6-/N-(2'-Methyl-4'-phenyl-1'(2'H)-isochinolinon-3-carbonyl)-2R-2-aminophenylacetamid/-2,2-dimethyl-penam-carbonsäure.

34. Verfahren zur Herstellung von Verbindungen nach Patentanspruch 1, dadurch g e k e n n z e i c h n e t , daß man eine Verbindung der Formel II

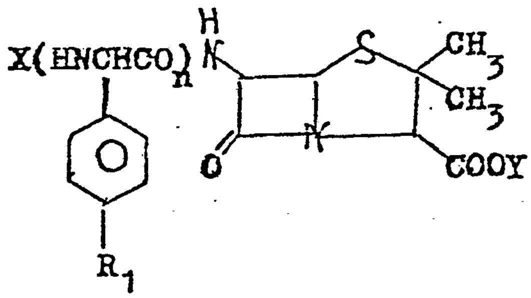

II

in der R und n die oben erwähnten Bedeutungen aufweisen, X Wasserstoff oder Trialkylsilylrest ist und Y ein Metallion, Ammonium oder einen Trialkylsilylrest bedeutet, mit Verbindungen der allgemeinen Formel

R - CO - Z          III

umsetzt, in welcher R die oben angeführten Bedeutungen aufweist, und Z -O-CO-Alk oder Halogen bedeutet.

0203216

Europäisches Patentamt

# EUROPÄISCHER RECHERCHENBERICHT

Nummer der Anmeldung

EP 85 10 6623

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 101, Nr. 3, 16. Juli 1984, Seite 573, Nr. 23181c, Columbus, Ohio, US; M. KHAIMOVA et al.: "Acylamine penam and cephem derivatives containing 1(2H)-isoquinolinone and 1H-2-benzopyran-1-one rings", & KHIM. GETEROTSIKL. SOEDIN. 1984, (3), 321-6 * Zusammenfassung * | 1-34 | C 07 D 499/76<br>C 07 D 499/68<br>A 61 K 31/43 |
| D,X | CHEMICAL ABSTRACTS, Band 90, Nr. 3, 15. Januar 1979, Seite 655, Nr. 23043p, Columbus, Ohio, US; & JP - A - 78 73 593 (MITSUBISHI CHEMICAL INDUSTRIES CO., LTD.) 30-06-1978 * Zusammenfassung * | 1,8,15,22,29,34 | |
| | | | RECHERCHIERTE SACHGEBIETE (Int. Cl.4) |
| Y | DE-A-2 809 594 (TIJIN LTD.) * Ansprüche * | 1,34 | |
| Y | US-A-4 159 268 (W.V. CURRAN) * Spalten 1,2 * | 1,34 | C 07 D 499/00<br>A 61 K 31/00 |
| Y | CHEMICAL ABSTRACTS, Band 92, Nr. 13, 31. März 1980, Seite 663, Nr. 111003x, Columbus, Ohio, US; & JP - A - 79 122 288 (AJINOMOTO CO., INC.) 21-09-1979 * Zusammenfassung * | 1,34 | |

Der vorliegende Recherchenbericht wurde für alle Patentansprüche erstellt.

| Recherchenort | Abschlußdatum der Recherche | Prüfer |
|---|---|---|
| DEN HAAG | 28-01-1986 | CHOULY J. |

KATEGORIE DER GENANNTEN DOKUMENTEN
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82